# EUROPEAN PATENT APPLICATION

(11) **EP 3 222 271 A1**
(43) Date of publication of application: **27.09.2017**
(21) Application number: 15860147.6
(22) Date of filing: 06.11.2015
(51) Int. Cl.: A61K 9/08, A61K 47/02, A61K 47/18, A61K 31/519

(54) **STABLE PHARMACEUTICAL COMPOSITION COMPRISING PEMETREXED OR PHARMACEUTICALLY ACCEPTABLE SALT THEREOF**

(30) Priority: 17.11.2014 KR 20140160225
(71) Applicant: Dong-A ST Co., Ltd., Seoul 130-823 (KR)
(72) Inventor: SOHN, Mi-Won, Yongin-si Gyeonggi-do 448-912 (KR); JANG, Sun-Woo, Seoul 140-764 (KR); WON, Dong-Han, Yongin-si Gyeonggi-do 449-718 (KR); KIM, Yong-Min, Yongin-si Gyeonggi-do 446-587 (KR); HWANG, Hyung-Don, Yongin-si Gyeonggi-do 446-905 (KR); MIN, Dong-Hun, Yongin-si Gyeonggi-do 446-905 (KR)
(74) Representative: Adam, Holger
(86) International application number: PCT/KR2015/011905
(87) International publication number: WO 2016/080687

(57) **Abstract**

The present invention relates to a composition of a ready-to-use injectable solution, comprising pemetrexed or pharmaceutically acceptable salts thereof, which comprises pemetrexed, sodium sulfite, and N-acetyl-L-cystein. With respect to use as an injectable preparation, the composition of a ready-to-use injectable solution comprising pemetrexed in accordance with the present invention is suitable for effective administration of pemetrexed since melting process of the main components before administration is not necessary, and the composition can be used by being diluted immediately in a perfusate. In addition, the properties of the composition do not change even in long-term storage, and pharmaceutical stability is significantly improved by inhibiting related substances below a standard, therefore the composition can be stored in a liquid preparation state.

## Description

### [Technical Field]

The present invention relates to a composition of a ready-to-use injection solution comprising pemetrexed as an active ingredient.

### [Background Art]

Pemetrexed is a 5-substituted pyrrolo[2,3-d]pyrimidine compound, having empirical formula of C₂₀H₂₁NC₅O₆ which is expressed in chemical formula 1 below, and used for treatment of non-small cell lung cancer as a multi-target antifolate drug with excellent antifolate effect.

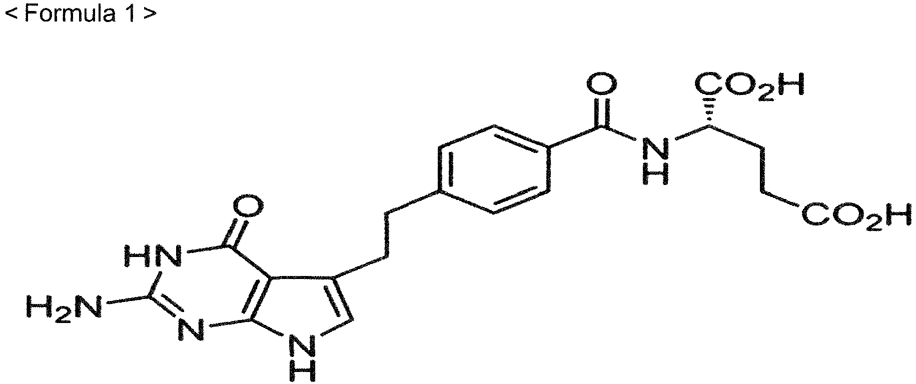

U.S. Patent No. 5,344,932 discloses preparation methods of antifolate salt derivatives including pemetrexed, and European Patent Application Publication No. 0434426 discloses a series of 4-hydroxypyrrolo[2,3-d]pyrimidine-L-glutamic acid derivatives.

Meanwhile, pemetrexed can be used in medicine as pharmaceutically acceptable salts thereof and, amongst them, pemetrexed disodium salt is marketed by Eli Lilly and Company as Alimta^{®} injection in dosage form of lyophilized injection. Alimta^{®} injection is administered to patient after being redissolved and diluted with 0.9% saline solution to an appropriate concentration.

Injections are classified into reconstituted injections, which can be used after dissolving and diluting powdered drug, and ready-to-use injections, which are immediately usable without the need for reconstitution. Lyophilized injections are reconstituted injections that can be used after redissolution and dilution processes. There is a concern of microbial exposure during those processes and it can be very dangerous to patient considering that the injection is an intravenous injection. Particularly in case of Alimta^{®} injection, there is also a concern of exposure to contamination during the redissolution and dilution processes by pharmacists, nurses and doctors, who are responsible for dispensing the cytotoxicity anticancer drug. Therefore it is necessary to develop a ready-to-use injection solution that is improved to be conveniently prepared and can prevent microbial contamination and be readily used.

Since the reconstituted Alimta^{®} injection has a stability problem of increase of related substances in the diluted solution over time when lyophilates of the injection are redissolved and diluted, it is usable for only a fixed period of time.

Also, the lyophilized injections, which are reconstituted injections, have the drawbacks of having a complicated process of preparing the lyophilizate, requiring special facilities and having an elongated preparation period. However, the ready-to-use injections can simplify the preparation process of the ready-to-use liquid injection solution and can be prepared with common facilities within short time.

International Publication No. WO 01/56575 discloses a pemetrexed-containing liquid injection comprising an antioxidant and pharmaceutically acceptable excipient. International Publication No. WO179310 discloses a pemetrexed-containing liquid injection comprising a stabilizer. Chinese Patent Application No. 1,907,284, Chinese Patent Application No. 101,081,301, European Patent Application No. 2,666,463, and European Patent Application No. 2,612,655 disclose stabilization of pemetrexed-containing liquid injections. However, the pemetrexed-containing liquid injection solutions specified in the patent application publications above are not proven to satisfy the standard of Alimta^{®} injection, which has the individual related substance within 0.2% and total related substance within 1.5%. Therefore, the solutions above are not proven to be safe as ready-to-use injection solutions.

In order to develop a pemetrexed-containing liquid injection as a ready-to-use injection that is administrable to an actual patient, a technology that can stably maintain the main component of the solution during storage period is needed. Particularly, with a single antioxidant that can be used in injection, it is difficult to secure sufficient stability of pemetrexed in the liquid state.

Hence, while the inventors of the present invention were conducting a study to develop the liquid composition of pemetrexed-containing injection, they found that the pemetrexed-containing liquid composition as a ready-to-use injection solution shows a very little change of property, no change in pH and maintains the stability of individual related substance, total related substances, and pemetrexed content during the storage period due to its markedly improved long-term storage stability. Thus the present invention has been achieved.

### [Disclosure]

### [Technical Problem]

An object of the present invention is to provide a stable composition of a ready-to-use injection solution comprising pemetrexed or salts thereof.

### [Technical Solution]

To achieve the above-mentioned object, the present invention provides a ready-to-use injection solution comprising pemetrexed, as the active ingredient, sodium sulfite, and N-acetyl-L-cysteine.

Hereinafter, the present invention will be described in detail.

To achieve the above-mentioned object, the composition of a ready-to-use injection solution should meet the following requirements:
First, the main component should be readily dissolved so that it can be readily used, and
Second, the stability of the composition, in which pemetrexed is dissolved, should be secured.

Stabilizers conventionally used for ready-to-use injection medications are sulfide compounds, EDTA, propylgallate, tocopherol, TPGS, amino acids, organic acids of ascorbic acids, citric acid, sulfite, cysteine, BHT, and BHA.

In the present invention, when one of the stabilizers selected from the above is added a liquid composition comprising pemetrexed, a clear change in color or increase of related substance is observed during storage.

In the present invention, the ready-to-use injection solution comprises pemetrexed as the active ingredient and sodium sulfite and N-acetyl-L-cysteine as the stabilizers.

In the present invention, sodium sulfite and N-acetyl-L-cysteine improve the stability of the active ingredient, pemetrexed or pharmaceutically acceptable salts thereof, by removing oxygen within the liquid composition through antioxidative activity. Particularly, unlike when single antioxidant is used, a change of color is not observed and increase rate of related substance is very small.

The liquid composition according to the present invention comprises pemetrexed or pharmaceutically acceptable salts thereof in a concentration of 1 to 100mg/mL, and preferably 10 to 80mg/mL. A ready-to-use injection, comprising liquid composition with the concentration of pemetrexed as low as 1 mg/mL or below, shows a big increase of related substance during storage. A ready-to-use injection, comprising liquid composition with the concentration of pemetrexed as high as 100mg/mL or over, cannot be prepared without special solubilization technique due to solubility limit of pemetrexed. Thus they are excluded from the present invention.

The liquid composition according to the present invention, therefore, may comprise pemetrexed and sodium sulfite in molar concentration ratio of 1:0.002∼0.1 and simultaneously comprise pemetrexed and N-acetyl-L-cysteine in molar concentration ratio of 1:0.05∼0.3. As shown in examples below, a ready-to-use injection solution, wherein molar concentration ratio of pemetrexed, sodium sulfite and N-acetyl-L-cysteine is 1:0.002∼0.1:0.05∼0.3, shows no change of properties and can suppress the formation of total and individual related substances to values below the standard. It can, also, maintain its storage stability with only a little change of content when stored even under the condition of 25°C, 60% RH and 40°C, 70% RH.

Accordingly, the present invention provides a ready-to-use injection comprising pemetrexed or pharmaceutically acceptable salts thereof, as the active ingredient, sodium sulfite and N-acetyl-L-cysteine, so that the injection has very outstanding storage stability and can be readily used.

Also, the above pharmaceutically acceptable salts of pemetrexed according to the present invention may be acid addition salts formed by pharmaceutically acceptable free acids. The above free acids may be inorganic and organic acids. The inorganic acids may be, but are not limited to, hydrochloric acid, bromic acid, sulfuric acid, sulfurous acid, and phosphoric acid. The organic acids may be, but are not limited to, citric acid, acetic acid, maleic acid, fumaric acid, glucosan, methane sulfonic acid, gluconic acid, succinic acid, tartaric acid, 4-toluene sulfonic acid, galacturonic acid, embonic acid, glutamic acid, and aspartic acid. Also, salts derived from appropriate bases may include, but are not limited to, alkali metals, such as sodium and potassium, or alkali earth metals, such as magnesium.

According to the present invention, racemates, optical isomers, polymorphs, hydrates, or solvates are collectively referred to as the above pemetrexed or pharmaceutically acceptable salts thereof. The above pemetrexed or pharmaceutically acceptable salts thereof in accordance with the present invention may include a stoichiometric or non-stoichiometric amount of water bound thereto by non-covalent intermolecular forces.

According to the present invention, the above composition of the ready-to-use pemetrexed-containing injection solution may be prepared with water for injection or 0.9% sodium chloride solution. The above composition of the ready-to-use pemetrexed-containing injection solution may also include pharmaceutically acceptable carriers, pH adjusters, and commonly used additives.

The ready-to-use pemetrexed-containing injection in accordance with the present invention is stable because there is no change of properties and very little related substance when it is stored in pH ranging from 6.0 to 8.5.

Also, according to the present invention, gas such as nitrogen or argon may be used to substitute oxygen in the vial when preparing the ready-to-use pemetrexed-containing injection. The ready-to-use pemetrexed-containing injection of the present invention may be provided using containers that common manufacturers of injection medications can consider, which may be, for example, glass vials, pre-filled injections, ampoules, glass bottle, and plastic bottle.

The composition of the present invention can be easily prepared through the following process:
1) Add and completely dissolve pH adjuster after dissolving excipients in water for injection.
2) Dissolve pemetrexed or pharmaceutically acceptable salts thereof in the above solution.
3) Fill a glass vial after sterilizing the solution by filtration.

### [Advantageous Effects]

The present invention provides the composition of the ready-to-use pemetrexed-containing injection solution. The composition stably maintains the active ingredient, pemetrexed or pharmaceutically acceptable salts thereof, with very little change of content during storage and provides injection preparation that has no change of properties and very little related substance.

Also, the pemetrexed-containing injection preparation in accordance with the present invention can be readily used thereby the convenience of administration is increased and the risk of microbial contamination that lyophilized medication products, which are reconstituted injection, may have can be prevented.

### [Best Mode]

Hereinafter, the present invention will be described in detail with embodiments. However, the embodiments and experimental examples below merely exemplify the present invention and do not limit the present invention thereto.

### <Embodiments 1 to 10> Preparation of the pemetrexed-containing ready-to-use injection according to the present invention

Thoroughly dissolve D-mannitol, sodium sulfite, and N-acetyl-L-cysteine specified in the table 1 below in 90mL of water for injection and apply 0.5N hydrochloric acid or 0.5N sodium hydroxide aqueous solution to adjust the solution to pH 7.0. After thoroughly dissolving the amount of pemetrexed specified in table 1 by slowly adding it to the solution, add water for injection to the solution to adjust its total volume to 100mL. Adjust pH of the solution to 7.5 and then filter it with 0.22um filter. After filling a glass vial with the solution, substitute oxygen within the vial with nitrogen and seal the vial. Designate the sealed vial as corresponding embodiment from 1 to 10.

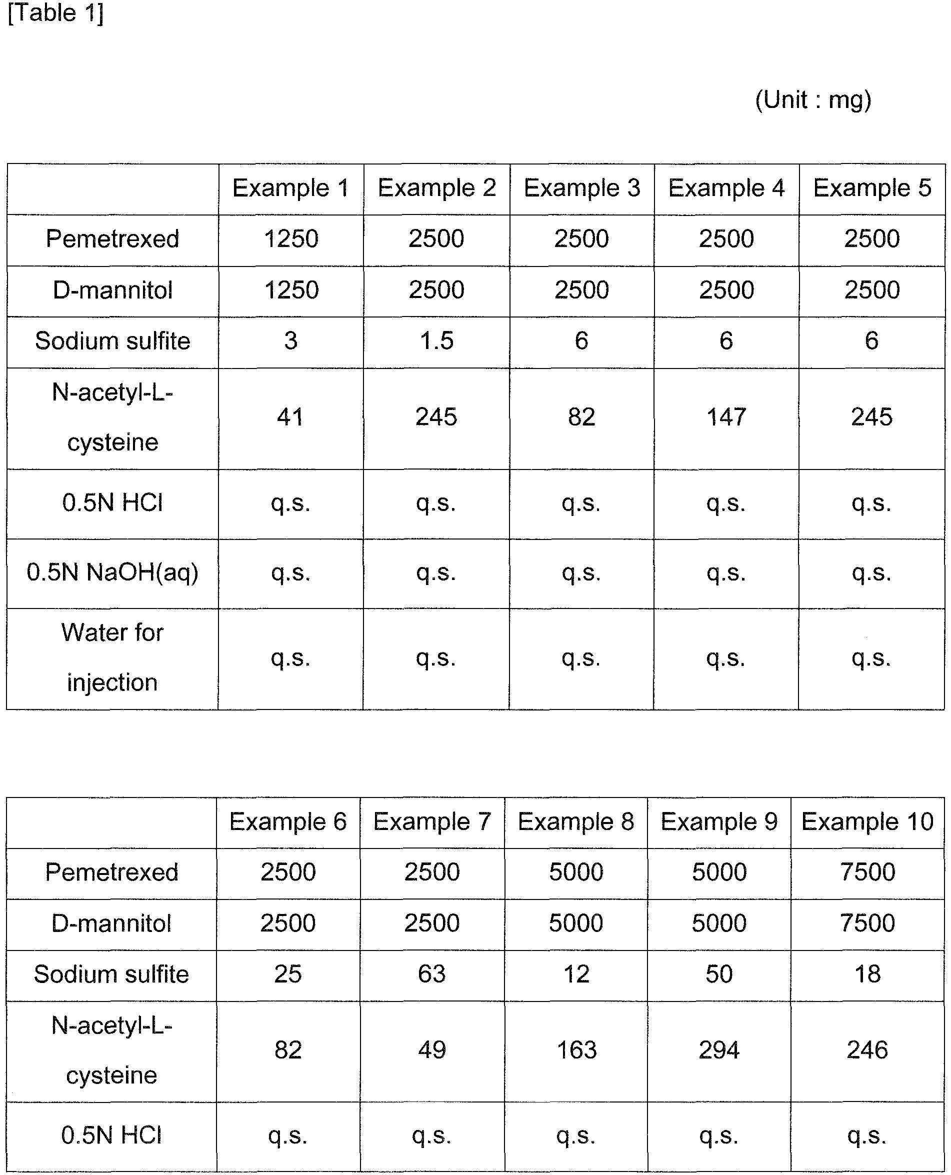

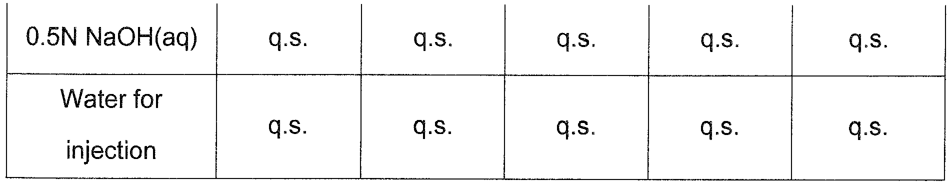

### <Embodiments 11 to 12> Preparation of the pemetrexed-containing ready-to-use injection according to the present invention

Thoroughly dissolve D-mannitol, sodium sulfite, and N-acetyl-L-cysteine specified in the table 2 below in 90mL of water for injection and apply 0.5N hydrochloric acid or 0.5N sodium hydroxide aqueous solution to adjust the solution to pH 7.0. After thoroughly dissolving the amount of pemetrexed specified in table 2 by slowly adding it to the solution, add water for injection to the solution to adjust its total volume to 100mL. Adjust each of the pH of the solution to pH 6.0 and pH 8.5 respectively and then filter it with 0.22um filter. After filling a glass vial with the solution, substitute oxygen within the vial with nitrogen and seal the vial. Designate the sealed vial as corresponding embodiment from 11 to 12.

**[Table 2]**

| | | (Unit: mg) |
|---|---|---|
| | Example 11 | Example 12 |
| Pemetrexed | 2500 | 2500 |
| D-mannitol | 2500 | 2500 |
| Sodium sulfite | 6 | 6 |
| N-acetyl-L-cysteine | 147 | 147 |
| 0.5N HCl | q.s. | q.s. |
| 0.5N NaOH(aq) | q.s. | q.s. |
| Water for injection | q.s. | q.s. |

### <Comparative Example 1 > Preparation of pemetrexed-containing liquid injection not comprising any stabilizer

Dissolve D-mannitol specified in the table 3 below in 90mL of water for injection and apply 0.5N hydrochloric acid or 0.5N sodium hydroxide aqueous solution to adjust the solution to pH 7.0. After thoroughly dissolving the amount of pemetrexed specified in table 3 by slowly adding it to the solution, add water for injection to the solution to adjust its total volume to 100mL. Adjust pH of the solution to 7.5 and then filter it with 0.22um filter. After filling a glass vial with the solution, substitute oxygen within the vial with nitrogen and seal the vial.

### <Comparative Examples 2 to 11 > Preparation of pemetrexed-containing liquid injection comprising conventional stabilizer

Thoroughly dissolve each D-mannitol, propyl gallate, sodium citrate hydrate, ascorbic acid, sodium bisulfite, disodium edetate hydrate, butylhydroxyanisole, butylhydroxytoluene, sodium thiosulfate hydrate, sodium pyrosulfite, and L-cysteine specified in the table 3 below in 90mL of water for injection and apply 0.5N hydrochloric acid or 0.5N sodium hydroxide aqueous solution to adjust the solution to pH 7.0. After thoroughly dissolving the amount of pemetrexed specified in table 3 by slowly adding it to the solution, add water for injection to the solution to adjust its total volume to 100mL. Adjust pH of the solution to 7.5 and then filter it with 0.22um filter. After filling a glass vial with the solution, substitute oxygen within the vial with nitrogen and seal the vial. Designate the sealed vial as corresponding comparative example from 2 to 11.

### <Comparative Examples 12 to 16> Preparation of pemetrexed-containing liquid injection comprising sodium sulfite alone

Thoroughly dissolve D-mannitol and sodium sulfite specified in the table 3 below in 90mL of water for injection and apply 0.5N hydrochloric acid or 0.5N sodium hydroxide aqueous solution to adjust the solution to pH 7.0. After thoroughly dissolving the amount of pemetrexed specified in table 3 by slowly adding it to the solution, add water for injection to the solution to adjust its total volume to 100mL. Adjust pH of the solution to 7.5 and then filter it with 0.22um filter. After filling a glass vial with the solution, substitute oxygen within the vial with nitrogen and seal the vial. Designate the sealed vial as corresponding comparative example from 12 to 16.

### <Comparative Examples 17 to 19> Preparation of pemetrexed-containing liquid injection comprising N-acetyl-L-cysteine alone

Thoroughly dissolve D-mannitol and N-acetyl-L-cysteine specified in the table 3 below in 90mL of water for injection and apply 0.5N hydrochloric acid or 0.5N sodium hydroxide aqueous solution to adjust the solution to pH 7.0. After thoroughly dissolving the amount of pemetrexed specified in table 3 by slowly adding it to the solution, add water for injection to the solution to adjust its total volume to 100mL. Adjust pH of the solution to 7.5 and then filter it with 0.22um filter. After filling a glass vial with the solution, substitute oxygen within the vial with nitrogen and seal the vial. Designate the sealed vial as corresponding comparative example from 17 to 19.

### <Comparative Example 20> Preparation of pemetrexed-containing liquid injection with different pH

Thoroughly dissolve D-mannitol, sodium sulfite, and N-acetyl-L-cysteine specified in the table 3 below in 90mL of water for injection and apply 0.5N hydrochloric acid or 0.5N sodium hydroxide aqueous solution to adjust the solution to pH 7.0. After thoroughly dissolving the amount of pemetrexed specified in table 3 by slowly adding it to the solution, add water for injection to the solution to adjust its total volume to 100mL. Adjust pH of the solution to 5.0 and then filter it with 0.22um filter. After filling a glass vial with the solution, substitute oxygen within the vial with nitrogen and seal the vial.

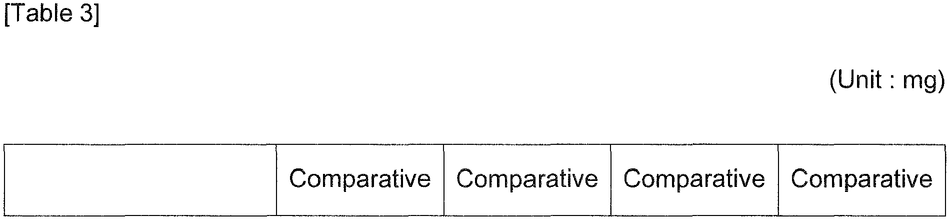

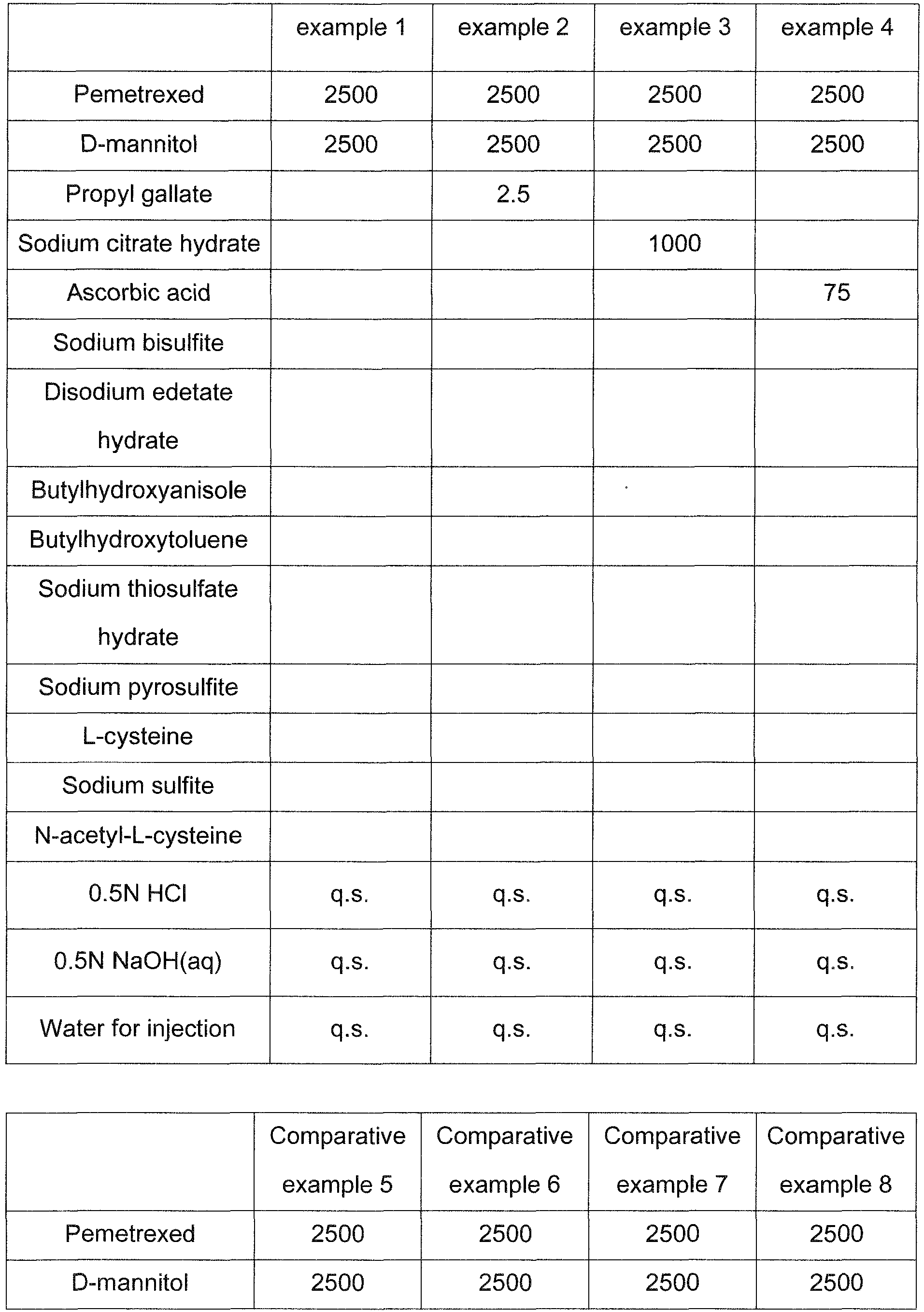

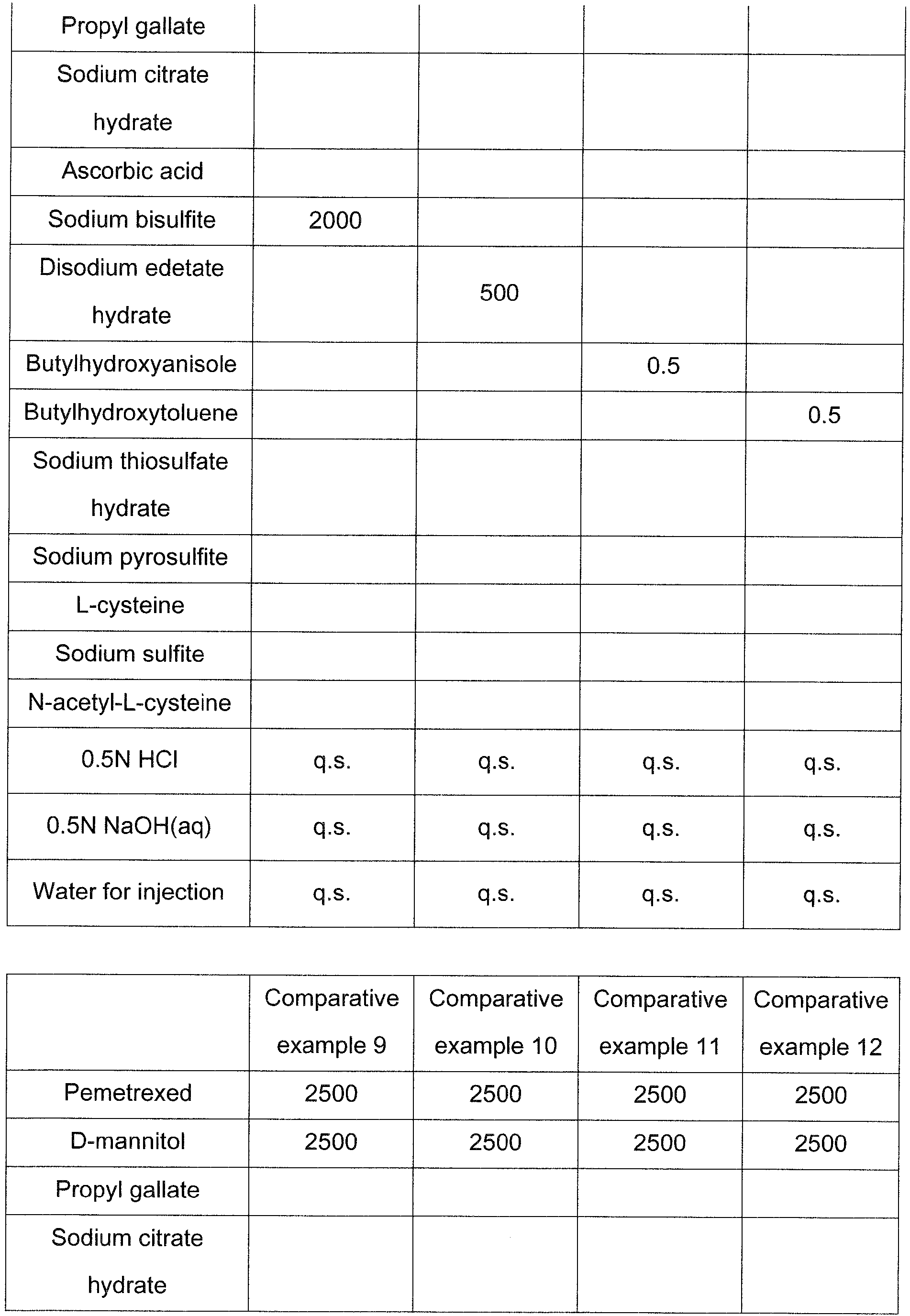

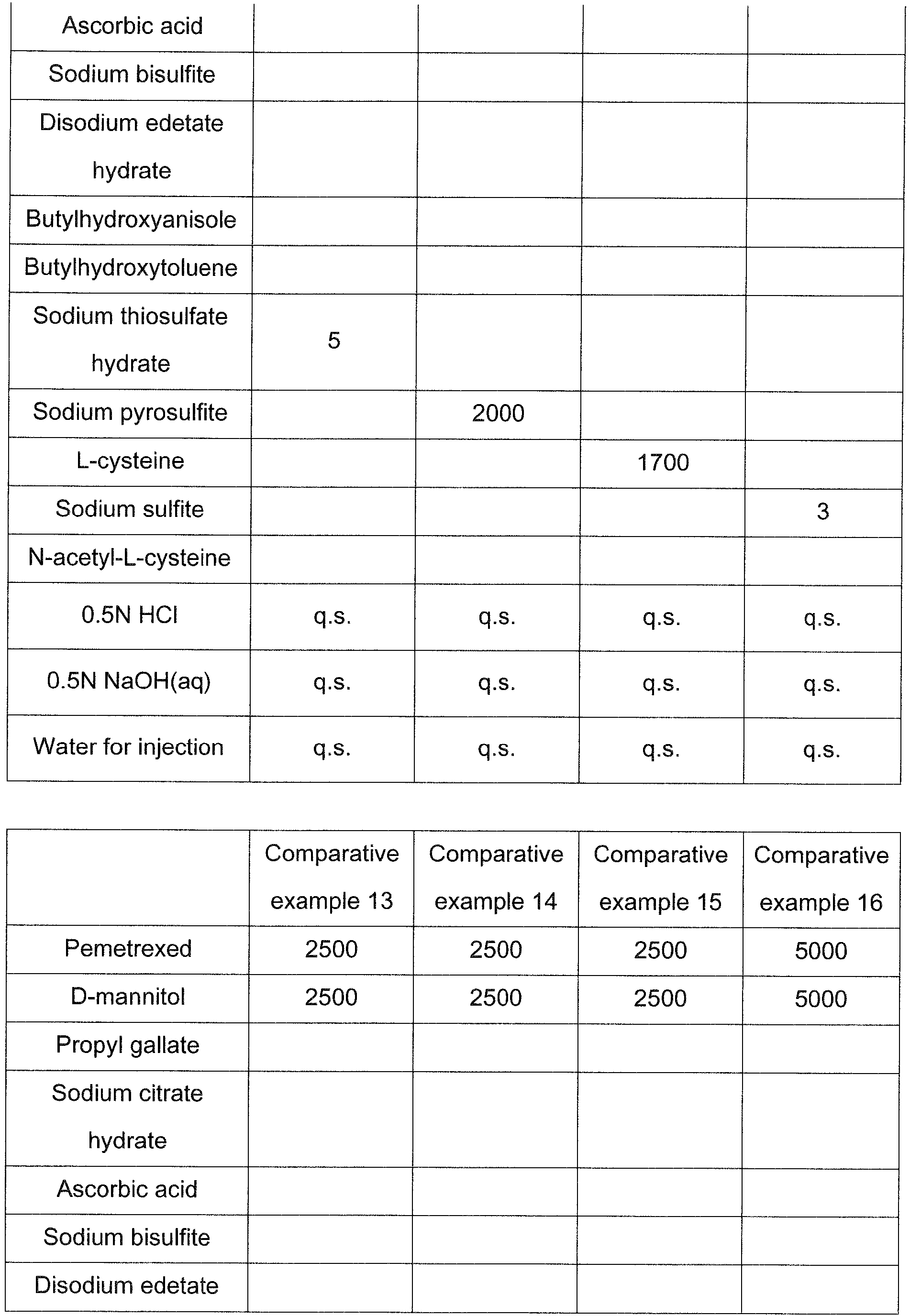

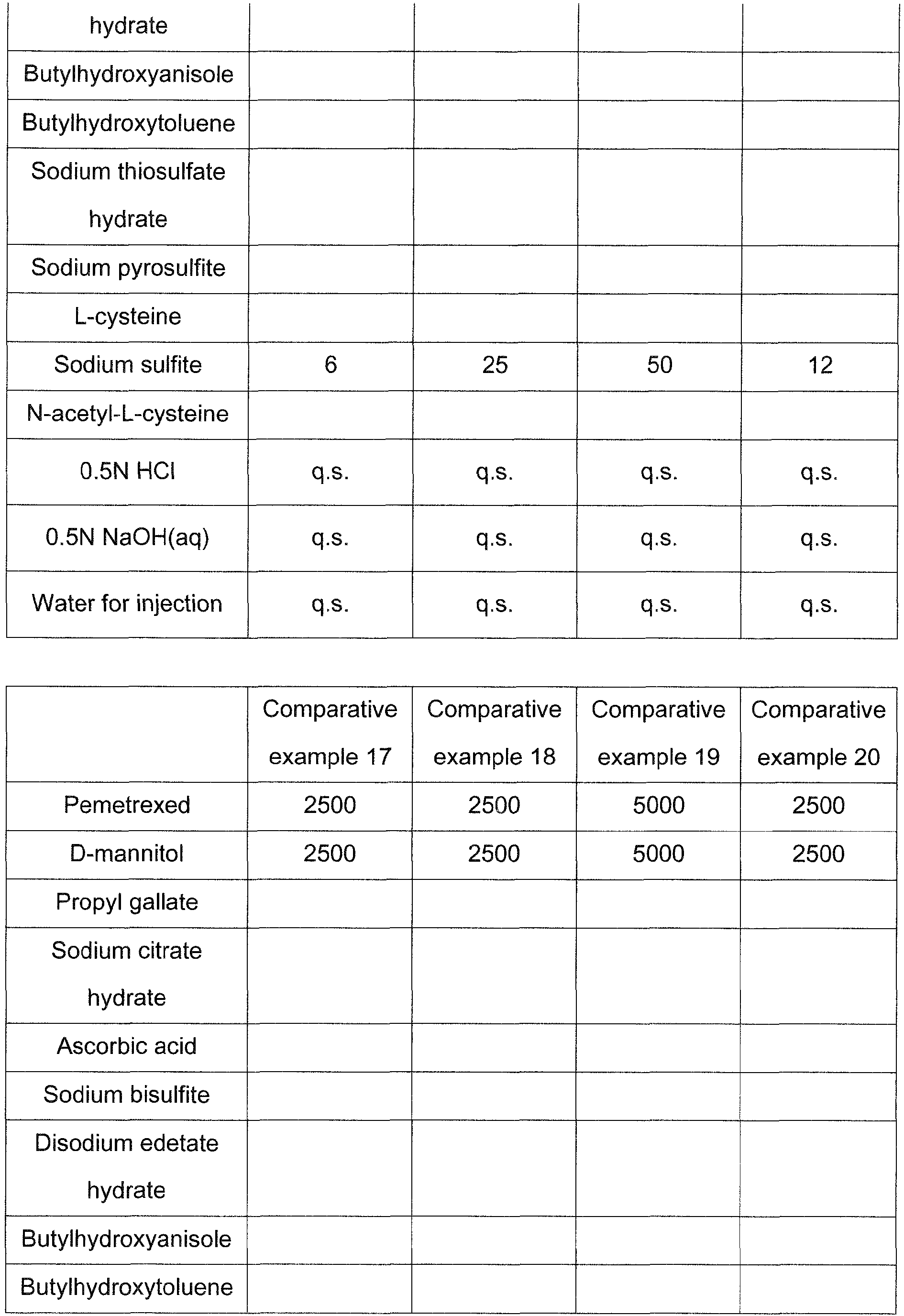

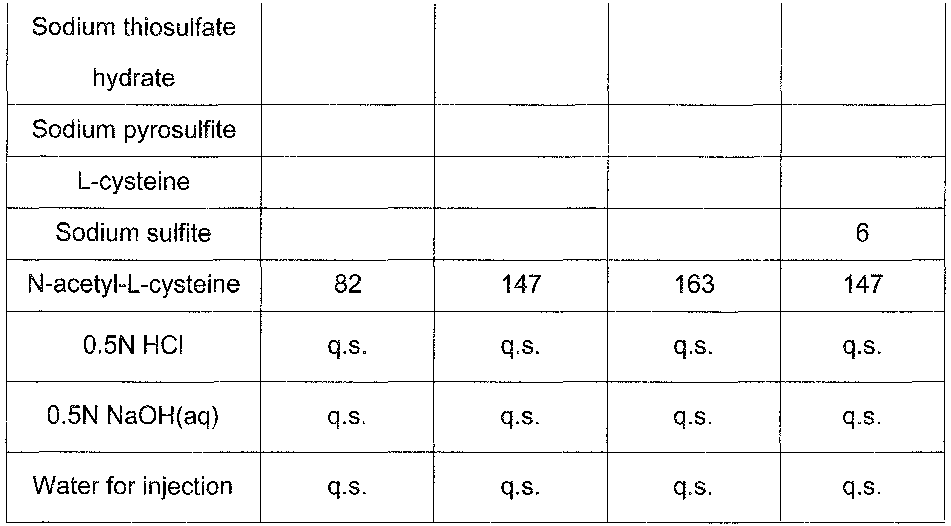

### <Experimental Example 1> Results of observation of change of property of pemetrexed-containing liquid injection

The property of the liquid injection is one of the important elements in deciding the quality of the product. Therefore, the properties of the ready-to-use pemetrexed-containing injections prepared in the above embodiments and pemetrexed-containing liquid injections prepared in the above comparative examples according to the present invention were observed after being stored in 40°C and 60°C conditions. The observations are shown in table 4.

**[Table 4]**

| | | Initial | 3 weeks, 60°C | 1 month, 40°C |
|---|---|---|---|---|
| Example | Stabilizer | | | |
| Example 1 | Sodium sulfite, N-acetyl-L-cysteine | clear, colorless | clear, colorless | clear, colorless |
| Example 2 | Sodium sulfite, N-acetyl-L-cysteine | clear, colorless | clear, colorless | clear, colorless |
| Example 3 | Sodium sulfite, N-acetyl-L-cysteine | clear, colorless | clear, colorless | clear, colorless |
| Example 4 | Sodium sulfite, N-acetyl-L-cysteine | clear, colorless | clear, colorless | clear, colorless |
| Example 5 | Sodium sulfite, N-acetyl-L-cysteine | clear, colorless | clear, colorless | clear, colorless |
| Example 6 | Sodium sulfite, N-acetyl-L-cysteine | clear, colorless | clear, colorless | clear, colorless |
| Example 7 | Sodium sulfite, N-acetyl-L-cysteine | clear, colorless | clear, colorless | clear, colorless |
| Example 8 | Sodium sulfite, N-acetyl-L-cysteine | clear, colorless | clear, colorless | clear, colorless |
| Example 9 | Sodium sulfite, N-acetyl-L-cysteine | clear, colorless | clear, colorless | clear, colorless |
| Example 10 | Sodium sulfite, N-acetyl-L-cysteine | clear, colorless | clear, colorless | clear, colorless |
| Example 11 | Sodium sulfite, N-acetyl-L-cysteine | clear, colorless | clear, colorless | clear, colorless |
| Example 12 | Sodium sulfite, N-acetyl-L-cysteine | clear, colorless | clear, colorless | clear, colorless |
| Comparative example 1 | - | clear, colorless | heavy yellowish green | yellowish green |
| Comparative example 2 | Propyl gallate | clear, colorless | yellow | yellow |
| Comparative example 3 | Sodium citrate hydrate | clear, colorless | yellow | yellow |
| Comparative example 4 | Ascorbic acid | clear, colorless | yellow | yellow |
| Comparative example 5 | Sodium bisulfite | clear, colorless | clear, colorless | clear, colorless |
| Comparative example 6 | Disodium edetate hydrate | clear, colorless | yellow | pale yellow |
| Comparative example 7 | Butylhydroxyanisole | clear, colorless | yellow | yellow |
| Comparative example 8 | Butylhydroxytoluene | clear, colorless | yellow | yellow |
| Comparative example 9 | Sodium thiosulfate hydrate | clear, colorless | pale yellow | clear, colorless |
| Comparative example 10 | Sodium pyrosulfite | clear, colorless | clear, colorless | clear, colorless |
| Comparative example 11 | L-cysteine | clear, colorless | yellow | pale yellow |
| Comparative example 12 | Sodium sulfite | clear, colorless | clear, colorless | clear, colorless |
| Comparative example 13 | Sodium sulfite | clear, colorless | clear, colorless | clear, colorless |
| Comparative example 14 | Sodium sulfite | clear, colorless | clear, colorless | clear, colorless |
| Comparative example 15 | Sodium sulfite | clear, colorless | clear, colorless | clear, colorless |
| Comparative example 16 | Sodium sulfite | clear, colorless | clear, colorless | clear, colorless |
| Comparative example 17 | N-acetyl-L-cysteine | clear, colorless | pale yellow | pale yellow |
| Comparative example 18 | N-acetyl-L-cysteine | clear, colorless | pale yellow | pale yellow |
| Comparative example 19 | N-acetyl-L-cysteine | clear, colorless | pale yellow | pale yellow |
| Comparative example 20 | Sodium sulfite, N-acetyl-L-cysteine | clear, colorless | clear, colorless | clear, colorless |

Pemetrexed-containing injection is clear and has colorless when it is prepared. However, as shown in table 4, comparative example 1, that does not comprise any stabilizer, shows the change of property after it is stored in 60°C for 3 weeks and 40°C for 1 month.

Also, most of the stabilizers used in the conventional ready-to-use injection medications show the change of property after being stored in 60°C for 3 weeks and 40°C for 1 month. In other words, comparative example 2 (propyl gallate), comparative example 3 (sodium citrate hydrate), comparative example 4 (ascorbic acid), comparative example 6 (disodium edentate hydrate), comparative example 7 (butylhydroxyanisole), comparative example 8 (butylhydroxytoluene), comparative example 11 (L-cysteine), and comparative examples 17 to 19 (N-acetyl-L-cysteine) show the change of property after being stored in 60°C for 3 weeks and 40°C for 1 month.

However, embodiments 1 to 12 (sodium sulfite + N-acetyl-L-cysteine), according to the present invention, do not show the change of property in the storage conditions mentioned above. Comparative example 5 (sodium bisulfite), comparative example10 (sodium pyrosulfite), comparative examples 12 to 16 (sodium sulfite), and comparative example 20 (sodium sulfite + N-acetyl-L-cysteine), also, do not show the change of property in the above storage conditions. Comparative example 9 (sodium thiosulfate hydrate), also, does not show the change of property after being stored in 40°C for 1 month.

Meanwhile, as shown in table 4, embodiments 1 to 12 in accordance with the present invention, ranging in pH from 6.0 to 8.5, and comparative example 20, which is prepared with the same composition of embodiment 4 but has the pH of 5.0, do not show any change of property. Also, among the other comparative examples with the same pH, some show the change of property and some do not, depending on the type of stabilizers. Therefore, it can be seen that the stability of property of liquid composition for pemetrexed-containing injection is not affected by pH. However, as in the present invention, the liquid compositions for the pemetrexed-containing injections ranging from pH 5.0 to 8.5 show stability in property when sodium sulfite and N-acetyl-L-cysteine are used in combination as the stabilizers of the compositions.

Therefore, it shows that the sodium sulfates or sodium sulfites are the stabilizers that do not change the property of the liquid composition of pemetrexed-containing injection.

Also, comparative examples 17 to 19(N-acetyl-L-cysteine), which use N-acetyl-L-cystein alone, show the change of property, whereas, embodiments 1 to 12 according to the present invention, which comprise sodium sulfite and N-acetyl-L-cysteine in combination as the stabilizers, show no change of property, which is an unprecedented phenomenon.

### <Experimental Example 2> Results of evaluation of the stability of total related substances in pemetrexed-containing liquid injection

The pemetrexed-containing ready-to-use injections, prepared in the above embodiments according to the present invention, and the pemetrexed-containing liquid injection, prepared in comparative examples, are stored in the 60°C and 40°C conditions. The total related substances are measured and shown in table 5. The results are evaluated using the commercially available Alimta^{®} injection as the standard, which has the total related substances within 1.5%.

**[Table 5]**

| Example | Stabilizer | Initial Total related substance (%) | 3 weeks, 60°C Total related substance(%) | 3 month, 40° C Total related substance( %) |
|---|---|---|---|---|
| Example 1 | Sodium sulfite, N-acetyl-L-cysteine | 0.37 | 0.91 | 0.92 |
| Example 2 | Sodium sulfite, N-acetyl-L-cysteine | 0.28 | 0.56 | 0.54 |
| Example 3 | Sodium sulfite, N-acetyl-L-cysteine | 0.05 | 0.34 | 0.64 |
| Example 4 | Sodium sulfite, N-acetyl-L-cysteine | 0.58 | 0.56 | 0.49 |
| Example 5 | Sodium sulfite, N-acetyl-L-cysteine | 0.15 | 0.63 | 0.92 |
| Example 6 | Sodium sulfite, N-acetyl-L-cysteine | 0.44 | 0.72 | 1.16 |
| Example 7 | Sodium sulfite, N-acetyl-L-cysteine | 0.49 | 0.85 | 0.97 |
| Example 8 | Sodium sulfite, N-acetyl-L-cysteine | 0.32 | 0.38 | 0.92 |
| Example 9 | Sodium sulfite, N-acetyl-L-cysteine | 0.56 | 0.64 | 0.98 |
| Example 10 | Sodium sulfite, N-acetyl-L-cysteine | 0.22 | 0.32 | 0.68 |
| Example 11 | Sodium sulfite, N-acetyl-L-cysteine | 0.47 | 0.58 | 0.75 |
| Example 12 | Sodium sulfite, N-acetyl-L-cysteine | 0.49 | 0.61 | 0.71 |
| Comparative example 1 | - | 0.31 | 2.47 | 1.58 |
| Comparative example 2 | Propyl gallate | 0.55 | 1.99 | 2.31 |
| Comparative example 3 | Sodium citrate hydrate | 0.35 | 0.93 | 1.93 |
| Comparative example 4 | Ascorbic acid | 0.54 | 2.14 | 2.45 |
| Comparative example 5 | Sodium bisulfite | 1.24 | 1.58 | 2.66 |
| Comparative example 6 | Disodium edetate hydrate | 0.87 | 1.54 | 2.46 |
| Comparative example 7 | Butylhydroxyanisole | 0.41 | 1.78 | 2.13 |
| Comparative example 8 | Butylhydroxytoluene | 0.49 | 1.85 | 1.97 |
| Comparative example 9 | Sodium thiosulfate hydrate | 0.56 | 1.37 | 1.74 |
| Comparative example 10 | Sodium pyrosulfite | 0.81 | 0.92 | 1.88 |
| Comparative example 11 | L-cysteine | 0.37 | 1.14 | 1.80 |
| Comparative example 12 | Sodium sulfite | 0.27 | 1.84 | 2.10 |
| Comparative example 13 | Sodium sulfite | 0.36 | 0.66 | 1.30 |
| Comparative example 14 | Sodium sulfite | 0.42 | 0.54 | 1.66 |
| Comparative example 15 | Sodium sulfite | 0.59 | 0.81 | 1.95 |
| Comparative example 16 | Sodium sulfite | 0.41 | 0.59 | 1.11 |
| Comparative example 17 | N-acetyl-L-cysteine | 0.33 | 1.55 | 1.24 |
| Comparative example 18 | N-acetyl-L-cysteine | 0.27 | 1.31 | 1.19 |
| Comparative example 19 | N-acetyl-L-cysteine | 0.32 | 1.20 | 0.94 |
| Comparative example 20 | Sodium sulfite, N-acetyl-L-cysteine | 0.65 | 0.90 | 1.01 |

If the measured total related substances of a preparation is within 1.5% in the 60°C condition, which is a harsh heat condition, the preparation is stable in high temperature. Therefore, this means that the preparation can be stably stored under the long-term storage condition (25°C 60%RH) and accelerated storage condition (40° C 70%RH) for a long period of time.

As shown in the table 5, the total related substances of comparative example 1, which is the liquid injection that does not comprise any stabilizer, is above the standard 1.5%. Therefore pemetrexed-containing liquid injections need to contain the stabilizers to prevent the formation of related substances.

All of the total related substances measured in embodiments 1 to 12(sodium sulfite and N-acetyl-L-cysteine), in accordance with the present invention, are below 1.5%.

However, amongst the comparative examples, comparative examples 17 to 19 (N-acetyl-L-cysteine), which show the change of property after being stored in 60°C for 3 weeks and 40°C for 1 month, show contrasting results from each other on the measurement of the total related substances. The total related substances of comparative examples 18 to 19(N-acetyl-L-cysteine) are below 1.5%, but that of comparative example 17(N-acetyl-L-cysteine) is above 1.5% of the total related substances. In other words, the formation of related substances of pemetrexed is variably inhibited depending on the concentration of N-acetyl-L-cysteine, but the change of property of pemetrexed within the solution with the same concentration of N-acetyl-L-cysteine is not inhibited.

Also, when sodium sulfite (comparative example 5), sodium thiosulfate (comparative example 9), sodium pyrosulfite (comparative example 10), and anhydrous sodium sulfite (comparative examples 12 to 16) are used alone as stabilizers for the composition of pemetrexed-containing liquid injection, the change of property does not occur in all of the above compositions but the degree of total related substances formed are very different from each other. In other words, the total related substances of comparative examples 13 and 16 show below 1.5%, whereas the total related substances of comparative example 5 (sodium bisulfite), comparative example 9 (sodium thiosulfate), and comparative example 10 (sodium pyrosulfite) are above 1.5%.

Also, the total related substances of the stabilizers of the conventional ready-to-use injection preparation, which are comparative example 2 (propyl gallate), comparative example 3 (citric acid), comparative example 4 (ascorbic acid), comparative example 6 (sodium edetate), comparative example 7 (butylhydroxyanisole), and comparative example 8 (butylhydroxytoluene), are above 1.5%.

Meanwhile, the total related substances of comparative example 20, which is prepared with the same composition as embodiment 4 in accordance with the present invention and adjusted to pH 5.0, is higher than the standard in contrast with the results of the present invention. Therefore, although the relationship between pH and formation of the related substances is not clearly investigated, the pemetrexed-containing liquid compositions according to the present invention are in the range of pH 6.0 to 8.5.

Therefore, using sodium sulfite and N-acetyl-L-cysteine in combination is necessary to inhibit the formation of the total related substances of pemetrexed-containing liquid composition. The composition of the ready-to-use pemetrexed-containing injection solution according to the present invention can suppress the formation of the total related substances within the standard value in the range of pH 6.0 to 8.5.

The embodiments, according to the present invention, as shown in experimental examples 1 and 2 above, show favorable degree of change of property and form the total related substances within the standard. Hereinafter, comparative examples 12 to 16, which show contrasting amount of the total related substances to each other, are compared with embodiments in accordance with the present invention to verify the degree of the formation of the individual related substance and evaluate the stability of the individual related substance of the solutions with varying pemetrexed to sodium sulfite ratio.

### <Experimental Example 3> Evaluation of stability of individual related substance of pemetrexed-containing liquid injection

The individual related substances of the pemetrexed liquid preparations prepared in the above embodiments and comparative examples stored in 60 °C and 4 °C conditions are measured and shown in table 6. The results are evaluated using the individual related substance of the commercially available Alimta^{®} injection as the standard, which is within 0.2%.

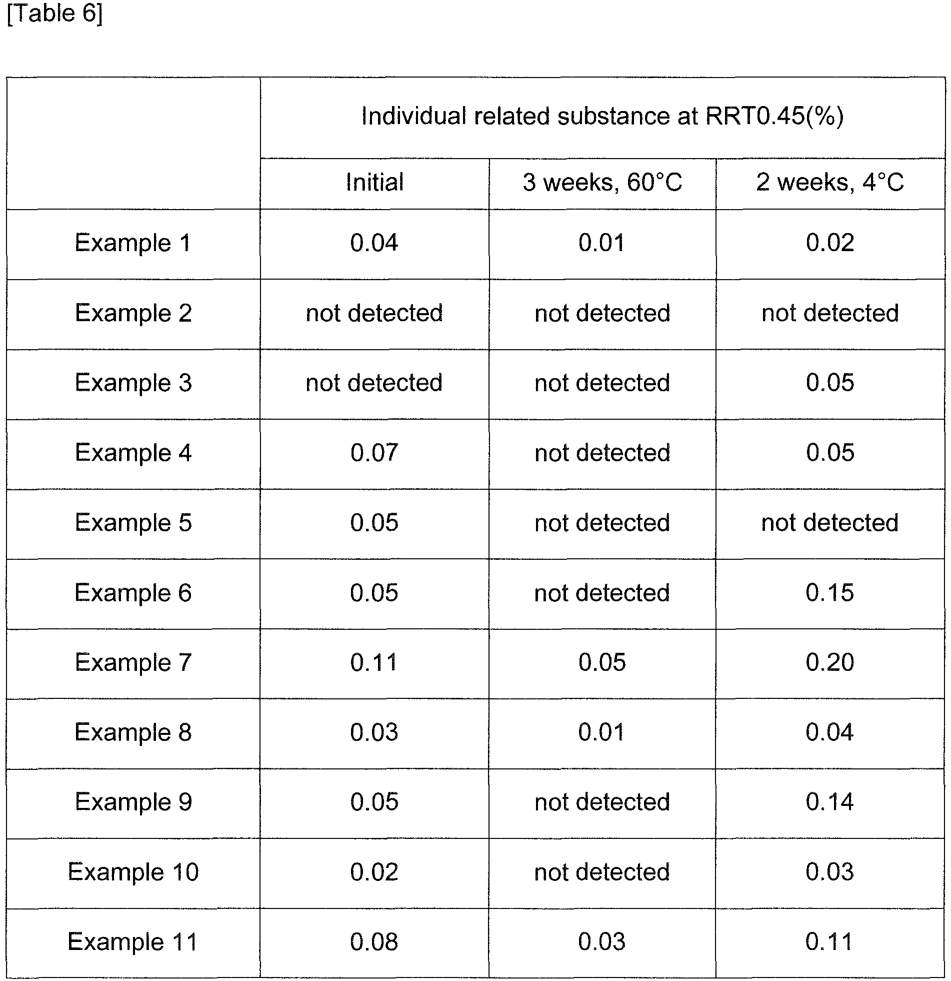

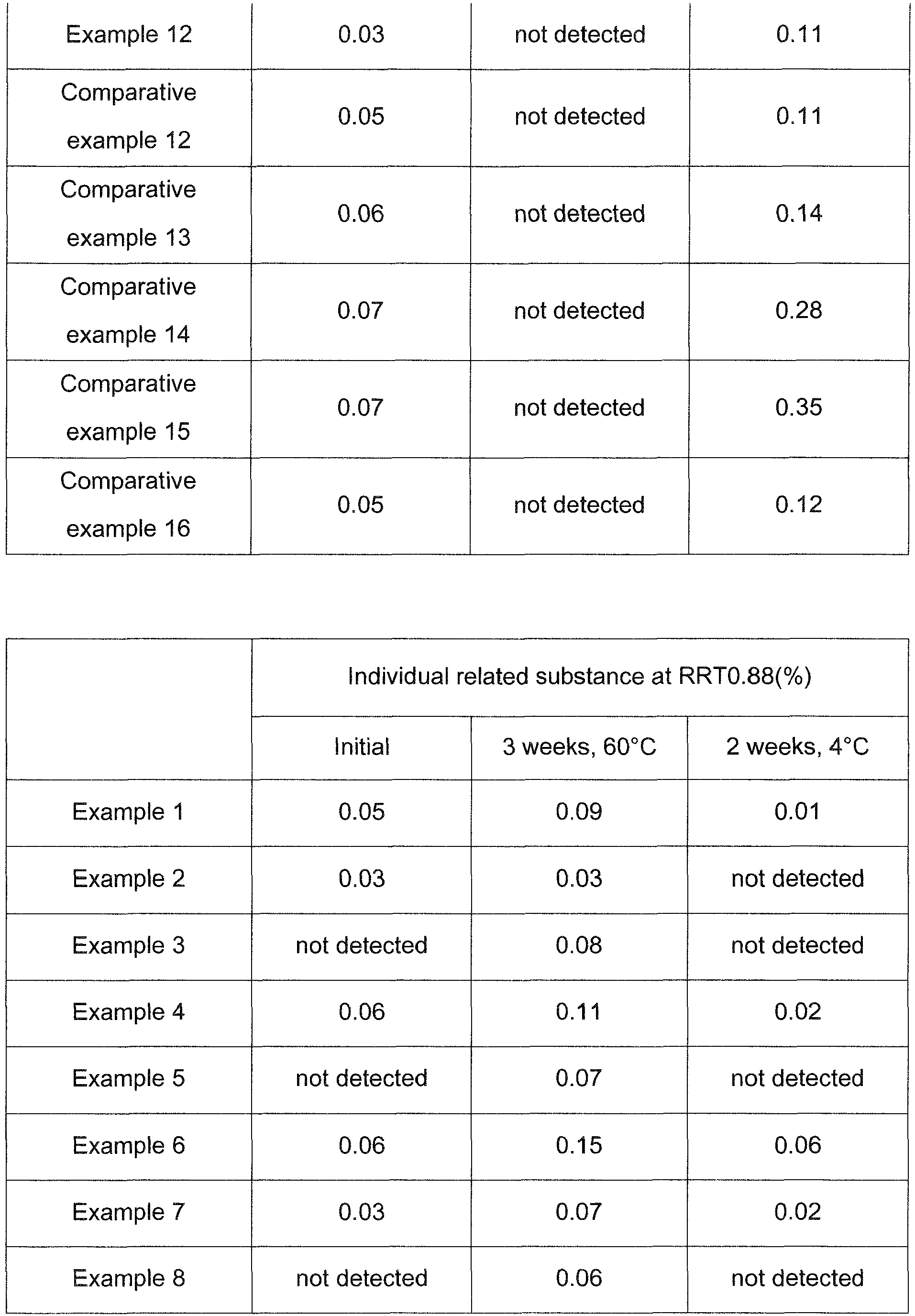

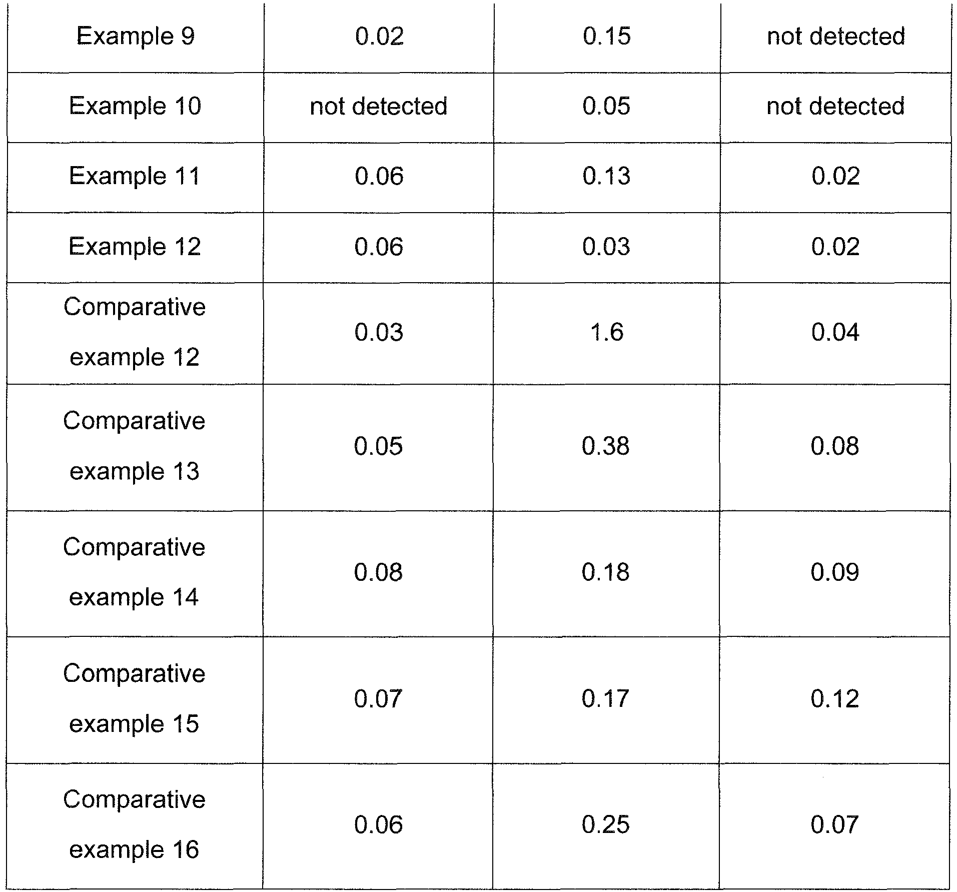

As shown in the table 6, the pemetrexed-containing liquid injections show different patterns of formation of the individual related substance depending on the concentration of the sodium sulfite.

In other words, when the ratios of pemetrexed : sodium sulfite of comparative examples 12, 13, and 16 are below 1:0.008, the individual related substance at RRT0.88 sharply increase in the 60°C condition. However, even though the molar concentration ratios of pemetrexed : sodium sulfite of embodiments 1 to 5, 8, and 10 to 12, in accordance with the present invention, are below 1:0.008 and, in particular, that of embodiment 2 is 1:0.002, the stability during the storage is maintained, unlike the comparative examples above, because sodium sulfite and N-acetyl-L-cysteine are used in combination. Therefore, the stabilities of the embodiments of the present invention are maintained during the storage when the ratios of pemetrexed : sodium sulfite are 1:0.002 or above. The molar concentration ratio of pemetrexed : N-acetyl-L-cysteine of the above solutions is 1:0.05∼0.3.

Also, as shown in comparative examples 14 and 15, when the ratio of pemetrexed : sodium sulfite is above 1:0.034, the individual related substance at RRT0.45 sharply increases in 4 °C condition and exceeds 0.2%, which is the standard value used to determine the stability of the ready-to-use pemetrexed-containing injection. However, even though the molar concentration ratios of pemetrexed : sodium sulfite of embodiments 6, 7, and 9, in accordance with the present invention, are 1:0.034 or more and, in particular, the molar concentration ratio of pemetrexed : sodium sulfite of embodiment 7 is 1:0.085, the stability during the storage is maintained, unlike the comparative examples above, because sodium sulfite and N-acetyl-L-cysteine are used in combination. Therefore, even if the ratio of pemetrexed : sodium sulfite is 1:0.085, the stability of the embodiment of the present invention is maintained during the storage. The molar concentration ratio of pemetrexed : N-acetyl-L-cysteine of the above solutions is 1:0.05∼0.3.

Therefore, in order to stabilize the individual related substance of the composition of the ready-to-use pemetrexed-containing injection solution, sodium sulfite and N-acetyl-L-cysteine need to be used in combination. As shown in the experimental examples above, when the ratio of pemetrexed : sodium sulfite : N-acetyl-L-cysteine is 1:0.002∼0.1:0.05∼0.3, the individual related substance is controlled below the standard.

### <Experimental Example 4> The change of content and solubility during the storage of the pemetrexed-containing liquid injections

The changes of content of the ready-to-use pemetrexed-containing injections according to the embodiments of the present invention during the storage are evaluated. After the pemetrexed-containing injections prepared in embodiments 4, 11, and 12, in accordance with the present invention and pemetrexed-containing injections prepared in comparative examples 1 and 20 are stored under the storage conditions specified in the table 7 below, they measured with content analysis method. The results are shown in the table 7 below.

**[Table 7]**

| | Initial Content (%) | 3 months, 25°C Content (%) | 2 weeks, 4°C Content (%) |
|---|---|---|---|
| Example 4 | 100.5 | 100.4 | 100.5 |
| Example 11 | 99.6 | 100.1 | 99.9 |
| Example 12 | 100.3 | 100.3 | 100.3 |
| Comparative example 1 | 99.8 | 98.7 | 99.7 |
| Comparative example 20 | 100.5 | 90.1 | 75.6 |

As shown in the table 7, the contents of the products of embodiments 4, 11, and 12, in accordance with the present invention, during the storage are stably maintained. Whereas, comparative example 1 shows very little change under the 2 weeks, 4°C storage condition but shows about 1% change of the content under the 3 months, 25°C storage condition. Particularly, comparative example 20 shows about 10% decline of the content under 3 months, 25° storage condition and show even greater decline under the 2 weeks, 4°C storage condition.

Also, embodiments 4, 11, and 12, in accordance with the present invention, do not show any formation of precipitate during storage, whereas, the comparative example 20, which has the same composition as embodiment 4 but is adjusted to pH 5.0, shows precipitate.

Therefore, the ready-to-use pemetrexed-containing injection of the present invention does not change in contents and maintains stability of the solubility.

## Claims

1. A composition of a ready-to-use pemetrexed-containing injection solution comprising pemetrexed or pharmaceutically acceptable salts thereof, as active ingredient, sodium sulfite, and N-acetyl-L-cysteine.

2. The composition of a ready-to-use injection solution according to claim 1, wherein the molar concentration ratio of pemetrexed to sodium sulfite to N-acetyl-L-cysteine is 1:0.002∼0.1:0.05∼0.3.

3. The composition of a ready-to-use injection solution according to claim 1, wherein the pH of the composition is from 6.0 to 8.5.

4. A method of preparing a ready-to-use pemetrexed-containing injection comprising the following steps:
① dissolving an excipient comprising sodium sulfite and N-acetyl-L-cysteine in water for injection or saline solution and adjusting the pH of the solution to a value of 7.0;
② mixing and dissolving pemetrexed in the solution prepared in the above ① and adjusting the pH of the solution to a value from 6.0 to 8.5;
③ filtering the solution prepared in ②;
④ filling a vial with the solution prepared in ③ and substituting the nitrogen within the vial with oxygen.
